(19) **Europäisches Patentamt**
**European Patent Office**
**Office européen des brevets**

(11) **EP 3 790 015 A1**

(12) **EUROPEAN PATENT APPLICATION**

(43) Date of publication:
**10.03.2021 Bulletin 2021/10**

(21) Application number: **19195361.1**

(22) Date of filing: **04.09.2019**

(51) Int Cl.:
**G16H 10/20** (2018.01)  G16H 50/70 (2018.01)
**G16H 50/20** (2018.01)  G16H 10/60 (2018.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Siemens Healthcare GmbH**
**91052 Erlangen (DE)**

(72) Inventors:
• **Hartung, Ulrich**
  **91094 Langensendelbach (DE)**
• **Schwemmer, Chris**
  **91301 Forchheim (DE)**
• **Sharma, Puneet**
  **Princeton Junction, 08550 (US)**

(54) **SYSTEM AND METHOD FOR AUTOMATED TRACKING AND QUANTIFICATION OF THE CLINICAL VALUE OF A RADIOLOGY EXAM**

(57) The invention relates to a method of and system for establishing a value of a clinical test and a corresponding medical imaging system. A first diagnosis of the clinical test is provided and at least one second diagnosis of the same type of diagnosis and for the same subject is fetched. An outcome of the clinical test is calculated by comparing the first diagnosis with the at least one second diagnosis. The value of the clinical test is determined by assessing the outcome of the clinical test in view of a cost of the clinical test.

## FIG 3

EP 3 790 015 A1

**Description**

The present invention relates to a method of and system for establishing a value of a clinical test and a corresponding medical imaging system.

In a fee-for-value reimbursement paradigm, there is increased focus on determining the value of a particular clinical test. In particular, there has been increased scrutiny on the value of the imaging examinations. Value in this regard means the outcome of a clinical test, in particular an imaging examination, in relation to the cost of the examination.

For hospital administrators and payers, it is often quite straightforward to precisely calculate the cost of a clinical test (imaging examination). On the other hand, determining the outcome for radiology exams is often quite challenging. Outcome in this regard means the probability that the result of the clinical test is correct, i.e. the diagnosis based on the clinical test was correct. This is due to the fact that a large majority of the clinical tests like radiology examinations are diagnostic in nature, and their contribution to the final outcome for the subject is often reflected downstream in the subject's care pathway. As a result, there might be declining rates for reimbursement for imaging exams, because the value is not clearly tracked and quantified in a scan-specific manner.

In daily clinical practice, there is no established way to accurately establish the value of an individual clinical test like a radiology examination in an exam-specific and subject-specific manner. In today's clinical practice, the value of a specific type of clinical test (radiology examination) is usually established in clinical trials and/or controlled studies. Such methods typically involve a downstream economic analysis that takes into account the subject outcomes and the total cost incurred in these controlled setting of a trial.

The results from these controlled studies are not easily transferable to daily clinical practice, which often has a different target population, different reading and reporting workflow and different levels of expertise for all the people involved (radiologists, technologists etc.).

The present invention therefore provides a method of establishing a value of a clinical test according to claim 1 and a corresponding system as well as a medical imaging system according to the further independent claims. Further refinements and embodiments are subject of the dependent claims.

According to a first aspect of the present invention a computer-implemented method of establishing a value of a clinical test comprises the steps providing, fetching, calculating and determining. In the step of providing, a first diagnosis of the clinical test is provided. In the step of fetching, at least one second diagnosis of the same type of diagnosis as the first diagnosis and for the same subject as the first diagnosis is fetched. In the step of calculating, an outcome of the clinical test is calculated by comparing the first diagnosis with the at least one second diagnosis. In the step of determining, the value of the clinical test is determined by assessing the outcome of the clinical test in view of a cost of the clinical test.

According to a second aspect of the present invention a system for establishing a value of a clinical test is arranged and configured to execute the steps of the method according to the first aspect of the present invention.

According to a third aspect of the present invention a medical imaging system comprises the system according to the second aspect of the present invention arranged and configured to execute the steps of the method according to the first aspect of the present invention.

For the present invention it is assumed without loss of generality that the value of a clinical test is defined by a mathematical function, which relies on the clinical outcome (probability of the diagnosis) of the clinical test and the cost associated with the clinical test for the subject:

$$\text{Value} = \text{Outcome} \; / \; \text{Cost}$$

For the present invention, the general term diagnosis (as the identification of the nature and cause of a certain phenomenon) is understood as medical diagnosis. Medical diagnosis is the process of determining which disease or condition explains the subject's symptoms and signs. It is referred to as diagnosis with the medical context being implicit. The information required for the diagnosis is mainly collected from the clinical test performed for or rather on the subject. The diagnosis may be the mere determination whether a certain disease is present or not (e.g. a tumour or malignant cells are present or not).

The first diagnosis is based on the clinical test performed for/on the subject. The clinical test may be any kind of medical examination used to establish medical information or findings about a subject. The medical information or findings allow for deducing a diagnosis. For example, the clinical test may be a blood picture giving certain blood levels as medical information or findings. Further, the clinical test may be a screening for malignant cells giving an amount of found cells with predefined characteristics and the first diagnosis may be the deduction whether a malignant tumour is present or not or rather which diameter such malignant tumour has. In specific cases such as screening for a particular disease (e.g. low-dose Chest CT for lung cancer screening, mammography for breast cancer screening), the first diagnosis may be limited to the ascertainment of either presence or absence of a disease (e.g. a tumour).

The first diagnosis answers a certain clinical question (e.g. is there a stenosis or what is the percentage of stenosis in a cardiac vessel? etc.). The clinical question may be inferred from the first diagnosis. The first diagnosis may be deduced automatically or manually by a practitioner based on the medical information or findings provided by the clinical test.

The clinical test may be a medical imaging examination like a computed tomography angiography (CTA) or an echocardiography (ultrasound examination) of the Left Ventricle. Correspondingly, the first diagnosis may be a percentage of stenosis (blockage) of a coronary vessel or a Left Ventricular ejection fraction (insight into the pump function of the heart).

The second diagnosis may be based on a further clinical test of the same or another type of clinical test, which may be performed sometime after the clinical test on which the first diagnosis is based. The further clinical test may also have been performed before the clinical test on which the first diagnosis is based. The type of diagnosis must be the same type of diagnosis as for the first diagnosis. For example, the further medical test regarding the percentage or degree of stenosis derived from the CTA may be a cardiac catheterization (heart cath) or rather invasive coronary angiography examination performed in a catheterization laboratory (cath lab), where the stenosis is examined directly in the vessel via a catheter such that the percentage of stenosis, the second diagnosis, can be determined directly. Or the further clinical test regarding the echocardiography of the Left Ventricle may be a cardiac magnetic resonance imaging (MRI), where also the left ventricular function is assessed. There may be more than one second diagnosis fetched regarding one first diagnosis.

The second diagnosis may be deduced automatically or manually by a practitioner based on the medical information or findings provided by the further clinical test. However, the method for deducing the second diagnosis from the medical information or findings of the further clinical test is not relevant for the present invention.

Based on the first diagnosis and the at least one second diagnosis the outcome of the clinical test is calculated. The outcome of the clinical test according to the present invention is an expressiveness of the first diagnosis or rather the respective clinical test in view of a certain clinical question. Thereto, the first diagnosis is compared to the at least one second diagnosis. The at least one second diagnosis is used as verification for the first diagnosis. This may be done by simple subtraction or division. However, more complex algorithms may be used for comparing the first diagnosis with the at least one second diagnosis. For example, in case the first diagnosis is the degree of stenosis determined by CTA and the second diagnosis is the degree of stenosis (determined by a further clinical test like the heart cath) the outcome may be the absolute value of the quotient of the difference of the first diagnosis and the second diagnosis divided by the second diagnosis.

```
Outcome =
| (first diagnosis - second diagnosis) / second diagnosis |
```

In case there is more than one diagnosis used to calculate the outcome, a mean value of the second diagnoses may be computed and the outcome may be calculated as the absolute value of the quotient of the difference of first diagnosis and the mean value of the second diagnoses divided by the mean value of the second diagnoses.

```
Outcome =
| (first diagnosis - mean value of second diagnoses) / mean
value of second diagnoses |
```

The value of the clinical test is determined based on the calculated outcome of the clinical test and based on the costs for the clinical test. The value of the clinical test according to the present invention is based on the expressiveness of the clinical test or rather of the first diagnosis in view of a certain clinical question and the costs for the clinical test. The costs may include all expenses related to the clinical test like preparative medications, follow-up care etc. The value may be computed according to the following equation:

```
Value = Outcome / Cost
```

The system according to the second aspect of the present invention comprises means for executing the steps of the method according to the first aspect of the present invention. In particular, the system may be a computer system or a data processing system like a personal computer (PC), a laptop, a tablet, a server, a distributed system (e.g. cloud system) and the like.

The medical imaging system according to the third aspect of the present invention may comprise means for conducting at least one type of medical imaging examination on subjects like a computer tomography (CT) scanner, a magnetic resonance imaging (MRI) scanner and/or a sonography system.

With the present invention clinical tests can be assessed, compared and rated by algorithm supported comparison. In particular, the usefulness of a clinical test for a certain clinical question or rather the expressiveness of the first diagnosis regarding the certain clinical question based on the clinical test can be established. Thereby, the value of the clinical test indicates whether the clinical test is indicated for a certain clinical question or not. Almost every imaging examination is analysed by AI algorithms (either exclusively, or in conjunction with a radiologist (either concurrently at the time of reading or before/after that)). As a result, nearly every imaging examination is heavily quantified by these AI algorithms. Therefore, all the major and minor clinical findings, their respective severity, location, type, extent etc. are readily available. This AI-driven analysis may happen on the imaging device itself, on a separate workstation (either local or remote) or on a radiologists reading and reporting workplace. The present invention takes advantage of these available quantitative results to track and quantify the value of the imaging examinations.

According to a further refinement of the present invention the first diagnosis is provided by a practitioner, in particular by a radiologist, in the step of providing.

The practitioner deduces based on the findings of the clinical test the diagnosis. For example, a radiologist deduces from the images recorded with a CTA examination the percentage or degree of stenosis in a cardiac vessel of the subject. The practitioner/radiologist records the first diagnosis of the clinical test and optionally further findings in a report. Also the indicators, values, morphology etc. on which his diagnosis is based (narrative text in the report) may be recorded in the report.

Also the second diagnosis may be deduced in the same way.

A first diagnosis provided by a practitioner/radiologist provides for a very reliable diagnosis due to the high level of experience and knowledge of a person having received the necessary training for becoming a practitioner/radiologist.

According to a refinement of the present invention, the first diagnosis is provided by an artificial intelligence (AI) based algorithm in the step of providing.

The AI based algorithm is executed by an AI system that has been trained on deriving a diagnosis based on the results and/or findings of a clinical test. For example, an AI system like a neural network, NN, may have been trained on deriving a diagnosis based on subject information like a blood picture, vital signs and/or medical image data. For example, an automated algorithm based on AI can be used to derive the percentage or degree of stenosis of a cardiac vessel of the subject as the first diagnosis.

Also the second diagnosis may be deduced in the same way.

A first diagnosis derived by means of an AI based algorithm provides for a fast and reliable first diagnosis. In particular correlations unseen by a human practitioner may be discovered by the AI based algorithm and a respective first diagnosis may be automatically derived without human interaction.

According to a further refinement of the present invention the clinical test is a medical imaging examination, in particular a computer tomography (CT) examination or a magnetic resonance imaging (MRI) examination or a sonography examination.

The CT examination may be a CTA examination, the MRI examination may be a cardiac MRI and the sonography examination may be an echocardiography examination.

Medical imaging examinations provide for detailed and reliable medical information and findings upon which the first diagnosis and also the second diagnoses can be based.

According to a refinement of the present invention, the step of providing comprises the steps:

- Loading an image data set generated with the medical imaging examination.
- Deriving the first diagnosis by an automated image analysis, in particular by a computer aided diagnosis (CAD) algorithm.

In the step of loading the image data set, the image data set, which was generated by the medical imaging examination, is loaded or fetched. For example, the images of the coronary vessels of the subject generated with CTA are loaded.

In the step of deriving the first diagnosis by the automated image analysis, the first diagnosis is derived by the automated image analysis. For example, the percentage or degree of stenosis of the coronary vessels of the subject is automatically derived by the CAD algorithm from the loaded image data.

Also the second diagnosis may be deduced in the same way.

Automated deriving of the first diagnosis by means of an automated image analysis (CAD algorithm) provides for fast and reliable first diagnoses without necessary prior human deductions.

According to a refinement of the present invention the step of providing comprises the steps:

- Loading a report of the clinical test.
- Deriving the first diagnosis by an AI based text analysis algorithm.

In the step of loading the report, the report of the clinical test is loaded. The report may have been written by a human practitioner (e.g. radiologist) or automatically deduced from the findings or medical information of the clinical test (e.g. CAD algorithm).

In the step of deriving the first diagnosis by the AI based text analysis algorithm, the first diagnosis is derived by the AI based text analysis algorithm from the loaded report. The loaded report is analysed by the AI based text analysis algorithm (natural language processing algorithm as described in US Patent Application No. 16/382,358), which has been trained on extracting a diagnosis from a report. In addition to the narrative text in the report (e.g. radiology report of a radiologist) the actual first diagnosis is available for the further steps of the present invention. In instances where the diagnosis is not clearly reported in the report, use of natural language processing algorithms to extract it from the text of the report may be used. For example, a deep learning based text analysis algorithm may be used to classify particular findings as either being present or absent when provided with a report. In addition, the text analysis algorithms may also extract secondary diagnoses from the report, such as incidental findings. In clinical practice, such incidental findings may incur further downstream testing (thereby raising the cost) without affecting the outcome.

Also the second diagnosis may be deduced in the same way.

Automated deriving of the first diagnosis by means of an AI based text analysis algorithm provides for fast and reliable first diagnoses from existing reports.

According to a refinement of the present invention, the method further comprises the step storing the value. In the step of storing the value, the value of the clinical test including a type of clinical test and the type of diagnosis is stored.

The value of the clinical test including the type of clinical test and the type of diagnosis may be stored on a server or cloud-based system. The type of clinical test can directly be inferred from the clinical test.

The stored value of the clinical test including the type of clinical test and the type of diagnosis can be used later on for posterior assessment or future selection of appropriate clinical tests when confronted with a certain clinical question.

According to a refinement of the present invention, the method further comprises the step storing clinical data. In the step of storing the clinical data, the clinical data of a subject including diagnoses of the subject and optionally including clinical tests used to derive the respective diagnoses are stored. In the step of fetching, all stored diagnoses that are of the same type as the first diagnosis are fetched as second diagnoses.

The clinical data including the diagnoses of the subject and optionally including the respective clinical tests may be stored on a server or cloud-based system. The first diagnosis may also be one of the stored diagnoses.

With the stored clinical data of the subject the clinical tests performed for/on the subject can be assessed and/or future selection of clinical tests can be refined.

According to a refinement of the present invention the steps are executed for more than one subject all subjected to the same clinical test. In the step of calculating, the outcome of the clinical test is based on the outcomes of the more than one subject.

The first diagnoses of the subjects (e.g. percentage/degree of stenosis derived from CTA) are compared to the respective second diagnoses of the subjects (e.g. percentage/degree of stenosis found during heart cath). The outcome is then based on all the outcomes of the more than one subject. For example, the outcome may be calculated as mean value of all the outcomes of the more than one subject.

The more diagnoses of subjects who have been subjected to the same clinical test (e.g. CTA examination) are used to calculate the outcome, the more precise the outcome and the value of the clinical test will become.

According to a further refinement of the present invention the steps are executed for a cohort of subjects all subjected to the same clinical test and wherein in the step of calculating, the outcome of the clinical test is based on the outcomes of the cohort.

A cohort is a group of subjects, all having at least one common property like same age group (e.g. 39 to 49 years) same race, same gender, sequence of same genetic expressions, equal medical history and the like.

In case the diagnoses of subjects all belonging to a cohort are used the preciseness of the outcome or rather of the value of the clinical test can be increased even more.

According to a refinement of the present invention the method further comprises the subsequent step selecting. In the step of selecting, a clinical test for a subject is selected in view of a clinical question based on at least one stored value of a clinical test.

In case a practitioner has to select one of several different clinical tests for a subject in view of a certain clinical question, the stored value of each clinical test can be used to select the best clinical test, namely the clinical test with the highest stored value, for the present clinical question. The selecting of the clinical test may be done automatically or manually based on a provided choice of clinical tests with respective values.

By selecting the clinical test with the highest value in view of a certain clinical question unnecessary clinical tests can be avoided and, thus, the costs for optimal treatment of the subject can be reduced. Further, unnecessary harm to the patient like unnecessary x-ray doses due to x-ray examinations with low value for the respective clinical question can be avoided.

According to a refinement of the present invention the value is weighted with a pre-test probability and/or a post-test probability in the step of determining.

Pre-test probabilities, are assessments whether a certain disease is present before the (planed) clinical test7 imaging examination is conducted. The assessments are done by using published scoring systems while comparing the individual case with a well-defined and known cohort out of the published score. Thereby, the cohort and the respective outcome are known. The pre-test probability (before the clinical test) accounts for a probability of presence of a certain disease.

An clinical test or rather imaging examination should provide insight into the presence and stage of the disease which then is changing the post-test probability to either final diagnosis or again a new pre-test probability of presence of the disease for the next clinical test/imaging examination. The post-test probability (after the clinical test) accounts for the results of the clinical test.

By the additional weighting with the pre-test and/or post-test probability the outcome and value of the clinical test can be further refined.

According to a refinement of the present invention the value is weighted with a level of appropriateness of the clinical test in the step of determining.

The level of appropriateness may be acquired from the "Appropriate Use Criterion" guidelines for the clinical test or rather imaging examination. Appropriate Use Criteria are used while selecting a respective clinical test/imaging examination in order to check their meaningfulness. Out of the different levels "not appropriate", "might be appropriate", "could be appropriate" and "is appropriate" the comparison to the currently established clinical test selection process can be further improved while using the herein described value-based score system.

By the additional weighting with the level of appropriateness of the clinical test the outcome and value of the clinical test can be further refined.

According to a fourth aspect, the invention provides a computer program product comprising executable program code configured to, when executed, perform the method according to any embodiment of the first aspect of the present invention.

According to a fifth aspect, the invention provides a non-transitory computer-readable data storage medium comprising executable program code configured to, when executed, perform the method according to any embodiment of the first aspect of the present invention.

According to a sixth aspect, the invention provides a data stream comprising, or configured to generate, program code configured to, when executed, perform the method according to any embodiment of the first aspect of the present invention.

The present invention and its technical field are subsequently explained in further detail by exemplary embodiments shown in the drawings. The exemplary embodiments only conduce better understanding of the present invention and in no case are to be construed as limiting for the scope of the present invention. Particularly, it is possible to extract aspects of the subject-matter described in the figures and to combine it with other components and findings of the present description or figures, if not explicitly described differently. Equal reference signs refer to the same objects, such that explanations from other figures may be supplementally used.

Fig. 1 shows a schematic flow chart of the method of establishing a value of a clinical test.

Fig. 2 shows a schematic view of the system for establishing a value of a clinical test.

Fig. 3 shows a schematic view of the medical imaging system comprising the system for establishing a value of a clinical test.

Fig. 4 shows a schematic block diagram of a computer program product according to an embodiment of the fourth aspect of the present invention.

Fig. 5 shows a schematic block diagram of a data storage medium according to an embodiment of the fifth aspect of the present invention.

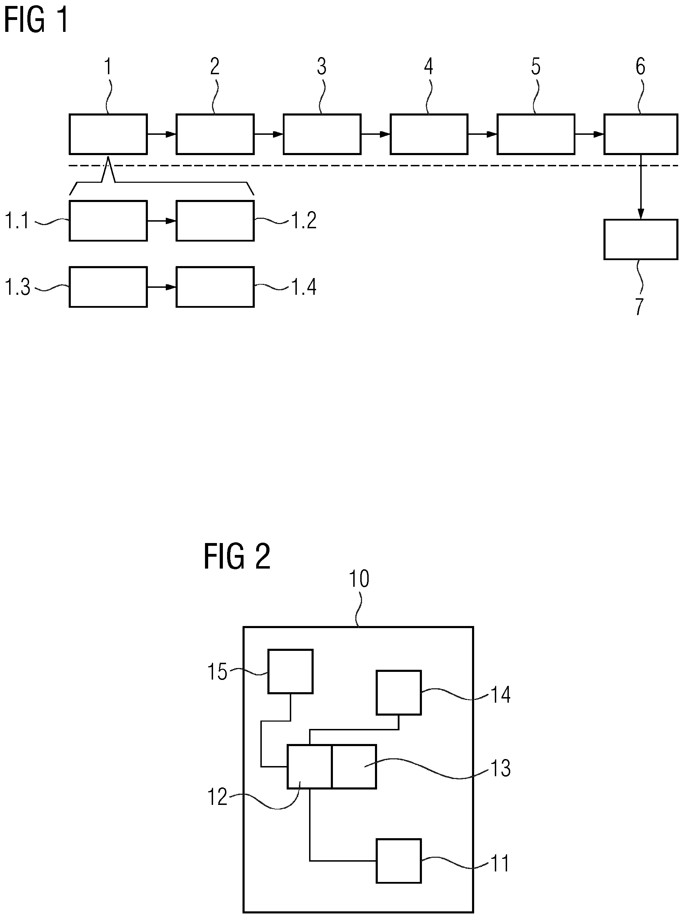

In Fig. 1 the computer-implemented method of establishing a value of a clinical test is schematically depicted. The method comprises the steps providing 1, fetching 2, calculating 3, determining 4, storing 5 the value and the step storing 6 clinical data as well as the subsequent step selecting 7. The steps 1 to 6 of the method may be executed in any reasonable sequence. The steps 1 to 6 of the method are executed for several subjects of at least one cohort. The subsequent step 7 of the method may be executed for at least one subject after the steps 1 to 6 have been executed for at least some of the subjects of the at least one cohort.

In the step of providing 1, the first diagnosis, which is a degree of stenosis, of the clinical test, which is a CTA examination, is automatically provided. Thereto, the step of providing 1 comprises the steps loading 1.1 an image data set and deriving 1.2 the first diagnosis by an automated image analysis.

In the step of loading 1.1 the image data set, the image data set generated with the CTA examination is loaded and in the step of deriving 1.2 the first diagnosis by an automated image analysis, the degree of stenosis is automatically derived by a computer aided diagnosis (CAD) algorithm.

Alternatively, the step of providing 1 comprises the steps loading 1.3 a report of the clinical test and deriving 1.4 the first diagnosis by an AI based text analysis algorithm.

In the step of loading 1.3 the report of the clinical test, the report of the CTA examination, which has been drawn up by a radiologist, is loaded and in the step of deriving 1.4 the first diagnosis by an AI based text analysis algorithm, the degree of stenosis is automatically derived by the AI based text analysis algorithm.

In the step of fetching 2, at least one second diagnosis of the same type of diagnosis as the first diagnosis and for the same subject as the first diagnosis is fetched. Here the second diagnosis is a degree of stenosis determined during heart cath of the respective subject.

In the step of storing 6 the clinical data, the clinical data of the subjects of the at least one cohort are stored. Here, the automatically derived first diagnoses (results of the CTA examination) and the fetched second diagnoses (results of the later heart cath) are stored together with the clinical tests (CTA examination and heart cath) for every subject of the at least one cohort. Here, the clinical data is stored on a cloud-based system.

In the step of calculating 3, the outcome of the CTA examination is calculated for every subject of the at least one cohort by the following equation:

$$\text{Outcome} =$$
$$| \text{ (degree of stenosis derived from CTA examination} - \text{degree of stenosis found during heart cath) / degree of stenosis found during heart cath } |$$

The outcome of the CTA examination is then calculated as the mean value of all the outcomes of all the subjects of the at least one cohort.

For example, a coronary CTA examination is performed earlier in the diagnostic phase, while an invasive coronary angiography examination is performed later in the final diagnosis/treatment phase. The findings of all the stenosis reported by the radiologist in the CTA examination are extracted from the respective radiology report by using a natural language processing algorithm like described in US Patent Application No. 16/382,358. This algorithm can extract the stenosis findings (such as the location and severity) in a structured table. When the invasive coronary angiography is performed at a later time, the same technique can be used to extract the stenosis findings from the invasive coronary angiography report. These two tables can then be automatically processed in order to compare the mismatch between two pairs of findings.

In another example, an echocardiography is able to compile a Left Ventricular Ejection fraction (insights into the pump function of the heart). Later there might be another examination by using cardiac MRI where also the left ventricular function is assessed. By the means of the present method (algorithm supported comparison) a match / mismatch can be calculated and made available to the practitioners.

In the step of determining (4), the value of the CTA examination in case of the clinical question, what degree of stenosis is present in the cardiac vessels of a subject, is determined by the following equation:

$$\text{Value} = \text{Outcome} / \text{Cost}$$

where the Cost is the cost of the CTA examination per subject. The clinical question can be inferred from the first and/or second diagnosis.

In the step of storing 5 the value, the value of the clinical test, here the CTA examination, is stored together with the type of clinical test (i.e. CTA examination) and the type of diagnosis (i.e. degree of stenosis of cardiac vessel). The type of diagnosis enables deriving the clinical question for which the value of the clinical test has been determined. The clinical data is stored on the cloud-based system.

With this method it is possible to analyse and track the clinical value of medical radiology examinations like CTA examination.

The steps 1 to 6 may be performed some time before the step 7 and also for different subjects (indicated by the dashed line in Fig. 1).

In the subsequent step of selecting 7, a clinical test for a subject (which may be a different subject than the subjects for which the first and second diagnoses have been provided/fetched) in view of a clinical question is selected based on at least one stored value of a clinical test. Here, for example, the CTA examination is automatically selected in view of the clinical question, what degree of stenosis is present in the cardiac vessels of the subject, in case the stored value for the CTA examination is higher than stored values for other possible clinical tests in view of said clinical question.

Thus, the optimal clinical test, namely the clinical test which will provide medical information or findings allowing for the most accurate diagnosis, is automatically selected.

In Fig. 2 the system 10 for establishing a value of a clinical test is schematically depicted. The system 10 comprises means, like a central processing unit, a storage etc., arranged and configured to execute the steps 1 to 7 of the method of Fig. 1. The system 10 is a data processing system and may be a personal computer (PC), a laptop, a tablet, a server, a distributed system (e.g. cloud system) and the like. The data processing system 10 comprises a central processing unit (CPU) 11, a memory having a random access memory (RAM) 12 and a non-volatile memory (MEM, e.g. hard disk) 13, a human interface device (HID, e.g. keyboard, mouse, touchscreen etc.) 14 and an output device (MON, e.g. monitor, printer, speaker, etc.) 15. The CPU 11, RAM 12, HID 14 and MON 15 are communicatively connected via a data bus. The RAM 12 and MEM 13 are communicatively connected via another data bus. The method according to the first aspect of the present invention and schematically depicted in Fig. 1 can be loaded in form of a computer program into the RAM 12 from the MEM 13 or another computer-readable medium having stored the respective computer program. According to the computer program the CPU executes the steps 1 to 7 of the method of Fig. 1. The execution can be initiated and controlled by a user (e.g. practitioner/radiologist) via the HID 14. The status and/or result of the executed computer program may be indicated to the user by the MON 15. The results of the executed computer program (first diagnoses, second diagnoses, outcomes and/or values of the clinical test) may be permanently stored on the non-volatile MEM 13 or another computer-readable medium. Based on the first and second diagnoses values of clinical tests (e.g. different types of CT/MRI/sonography examination) for given clinical questions are determined and stored in the MEM 13. The optimal clinical test for a given clinical question is automatically selected by the system 10 and may be output by the MON 15 and/or stored in the MEM 13.

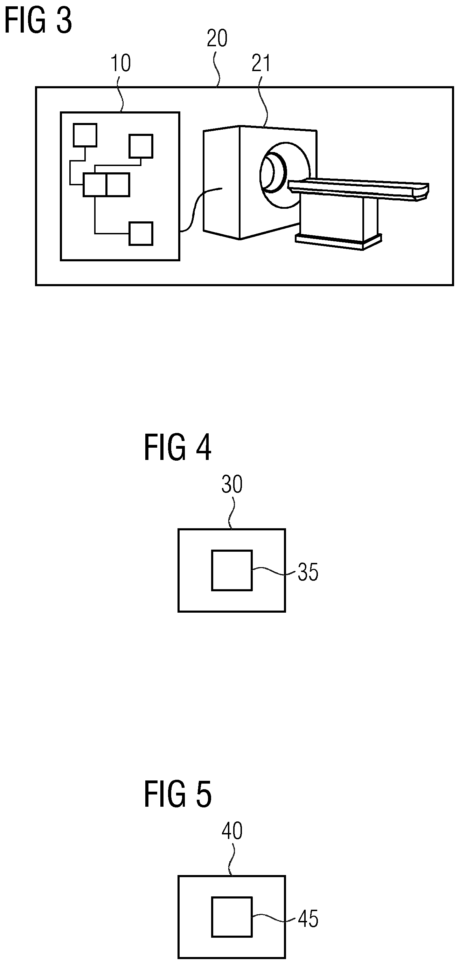

In Fig. 3 the medical imaging system 20 comprising the system 10 of Fig. 2 arranged and configured to execute the steps 1 to 7 of the method of Fig. 1 is schematically depicted. The medical imaging system comprises means for conducting a medical imaging examination like CT, MRI and/or sonography examination. Here, exemplarily, the medical imaging system comprises a CT scanner 21 arranged and configured to conduct at least CTA examinations. The CT scanner 21 is communicatively coupled to the system 10 and can receive the clinical test (here type of CT examination) that has been selected based on the value for the clinical test by the system 10. Based on the stored values of clinical tests (e.g. different types of CT/MRI/sonography examination) for a given clinical question, which have been derived and stored by the system 10, the optimal clinical test for the given clinical question is automatically selected and communicated to the CT scanner 21.

Fig. 4 shows a schematic block diagram of a computer program product 30 according to an embodiment of the fourth aspect of the present invention. The computer program product comprises executable program code 35 configured to, when executed, perform the method according to any embodiment of the first aspect of the present invention, in particular the method as it has been described with respect to the foregoing figures.

Fig. 5 shows a schematic block diagram of a non-transitory, computer-readable data storage medium 40 according to an embodiment of the fifth aspect of the present invention. The computer program product comprises executable program code 45 configured to, when executed, perform the method according to any embodiment of the first aspect of the present invention, in particular the method as it has been described with respect to the foregoing figures. The data storage medium 40 may comprise, or consist of, any type of computer memory, in particular semiconductor memory such as a solid-state memory. The data storage medium may also comprise, or consist of, a CD, a DVD, a Blu-Ray-Disc, an USB memory stick or the like.

Although specific embodiments have been illustrated and described herein, it will be appreciated by those of ordinary skill in the art that a variety of alternate and/or equivalent implementations exist. It should be appreciated that the exemplary embodiment or exemplary embodiments are only examples, and are not intended to limit the scope, applicability, or configuration in any way. Rather, the foregoing summary and detailed description will provide those skilled in the art with a convenient road map for implementing at least one exemplary embodiment, it being understood that various changes may be made in the function and arrangement of elements described in an exemplary embodiment without departing from the scope as set forth in the appended claims and their legal equivalents. Generally, this application is intended to cover any adaptations or variations of the specific embodiments discussed herein.

In the foregoing detailed description, various features are grouped together in one or more examples for the purpose of streamlining the disclosure. It is understood that the above description is intended to be illustrative, and not restrictive. It is intended to cover all alternatives, modifications and equivalents as may be included within the scope of the invention. Many other examples will be apparent to one skilled in the art upon reviewing the above specification.

Specific nomenclature used in the foregoing specification is used to provide a thorough understanding of the invention. However, it will be apparent to one skilled in the art in light of the specification provided herein that the specific details are not required in order to practice the invention. Thus, the foregoing descriptions of specific embodiments of the present invention are presented for purposes of illustration and description. They are not intended to be exhaustive or to limit the invention to the precise forms disclosed; obviously many modifications and variations are possible in view of the above teachings. The embodiments were chosen and described in order to best explain the principles of the invention and its practical applications, to thereby enable others skilled in the art to best utilize the invention and various embodiments with various modifications as are suited to the particular use contemplated. Throughout the specification, the terms "including" and "in which" are used as the plain-English equivalents of the respective terms "comprising" and "wherein," respectively. Moreover, the terms "first," "second," and "third," etc., are used merely as labels, and are not intended to impose numerical requirements on or to establish a certain ranking of importance of their objects. In the context of the present description and claims the conjunction "or" is to be understood as including ("and/or") and not exclusive ("either ... or") .

**Claims**

1. A computer-implemented method of establishing a value of a clinical test, comprising the steps:

   - providing (1) a first diagnosis of the clinical test;
   - fetching (2) at least one second diagnosis of the same type of diagnosis as the first diagnosis and for the same subject as the first diagnosis;
   - calculating (3) an outcome of the clinical test by comparing the first diagnosis with the at least one second diagnosis; and
   - determining (4) the value of the clinical test by assessing the outcome of the clinical test in view of a cost of the clinical test.

2. The method according to claim 1, wherein in the step of providing (1), the first diagnosis is provided by a practitioner, in particular by a radiologist.

3. The method according to claim 1, wherein in the step of providing (1), the first diagnosis is provided by an artificial intelligence, AI, based algorithm.

4. The method according to any of claims 1 to 3, wherein the clinical test is a medical imaging examination, in particular a computer tomography, CT, examination or a magnetic resonance imaging, MRI, examination or a sonography examination.

5. The method according to claim 4, wherein the step of providing (1) comprises the steps:

   - loading (1.1) an image data set generated with the medical imaging examination; and
   - deriving (1.2) the first diagnosis by an automated image analysis, in particular by a computer aided diagnosis, CAD, algorithm.

6. The method according to claim 3 or 4, wherein the step of providing (1) comprises the steps:

   - loading (1.3) a report of the clinical test; and
   - deriving (1.4) the first diagnosis by an AI based text analysis algorithm.

7. The method according to any of the preceding claim, further comprising the step:

   - storing (5) the value of the clinical test including a type clinical test and the type of diagnosis.

8. The method according to any of the preceding claim, further comprising the step:

   - storing (6) clinical data of a subject including diagnoses of the subject,

   wherein in the step fetching, all stored diagnoses that are of the same type as the first diagnosis are fetched as second diagnoses.

9. The method according to any of the preceding claim, wherein the steps are executed for more than one subject all subjected to the same clinical test and wherein in the step of calculating, the outcome of the clinical test is based on the outcomes of the more than one subject.

10. The method according to claim 9, wherein the steps are executed for a cohort of subjects all subjected to the same clinical test and wherein in the step of calculating, the outcome of the clinical test is based on the outcomes of the cohort.

11. The method according to any of claims 7 to 10, further comprising the subsequent step:

    - selecting (7) a clinical test for a subject in view of a clinical question based on at least one stored value of a clinical test.

12. The method according to any of the preceding claim, wherein in the step of determining (4), the value is weighted with a pre-test probability and/or a post-test probability.

13. The method according to any of the preceding claim, wherein in the step of determining (4), the value is weighted with a level of appropriateness of the clinical test.

14. A system (10) for establishing a value of a clinical test arranged and configured to execute the steps of the method according to any preceding claim.

15. A medical imaging system (20) comprising the system (10) according to claim 14 arranged and configured to execute the steps of the method according to any of claims 4 to 13.

FIG 1

FIG 2

FIG 3

FIG 4

FIG 5

Europäisches
Patentamt

European
Patent Office

Office européen
des brevets

# EUROPEAN SEARCH REPORT

Application Number

EP 19 19 5361

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/315505 A1 (ITU LUCIAN MIHAI [RO] ET AL) 1 November 2018 (2018-11-01) * the whole document * * in particular * * paragraphs 0005-0010, 0027-0035, 0060-0070; figures 4-6 * | 1-15 | INV. G16H10/20<br><br>ADD. G16H50/70 G16H50/20 G16H10/60 |
| X | WO 2012/131524 A2 (KONINKL PHILIPS ELECTRONICS NV [NL] ET AL.) 4 October 2012 (2012-10-04) * the whole document * * in particular * * page 1 - page 3; figure 2 * | 1-15 | |
| X | US 2014/019152 A1 (OP DEN BUIJS JORN [NL] ET AL) 16 January 2014 (2014-01-16) * the whole document * * in particular * * paragraph [0001] - paragraph [0027]; figures 2-9 * | 1-15 | |
| X | US 2008/171916 A1 (FEDER CARLOS [US] ET AL) 17 July 2008 (2008-07-17) * the whole document * * in particular * * paragraph [0022] - paragraph [0034]; figures 1-6 * * paragraph [0237] - paragraph [0296] * | 1-15 | **TECHNICAL FIELDS SEARCHED (IPC)**<br><br>G16H |
| X | WO 2012/122127 A2 (KEW GROUP LLC [US]; ELTON JEFFREY J [US] ET AL.) 13 September 2012 (2012-09-13) * paragraph [0005] - paragraph [0028] * * paragraph [0064] - paragraph [0074] * | 1-15 | |

The present search report has been drawn up for all claims

| Place of search | Date of completion of the search | Examiner |
|---|---|---|
| Munich | 14 February 2020 | Rákossy, Z |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**ANNEX TO THE EUROPEAN SEARCH REPORT
ON EUROPEAN PATENT APPLICATION NO.**

EP 19 19 5361

This annex lists the patent family members relating to the patent documents cited in the above-mentioned European search report.
The members are as contained in the European Patent Office EDP file on
The European Patent Office is in no way liable for these particulars which are merely given for the purpose of information.

14-02-2020

| Patent document cited in search report | | Publication date | Patent family member(s) | | Publication date |
|---|---|---|---|---|---|
| US 2018315505 | A1 | 01-11-2018 | NONE | | |
| WO 2012131524 | A2 | 04-10-2012 | CN | 103460213 A | 18-12-2013 |
| | | | EP | 2691895 A2 | 05-02-2014 |
| | | | JP | 6053749 B2 | 27-12-2016 |
| | | | JP | 2014515849 A | 03-07-2014 |
| | | | RU | 2013148017 A | 10-05-2015 |
| | | | US | 2014010432 A1 | 09-01-2014 |
| | | | WO | 2012131524 A2 | 04-10-2012 |
| US 2014019152 | A1 | 16-01-2014 | US | 2014019152 A1 | 16-01-2014 |
| | | | US | 2014019161 A1 | 16-01-2014 |
| US 2008171916 | A1 | 17-07-2008 | NONE | | |
| WO 2012122127 | A2 | 13-09-2012 | AU | 2012225666 A1 | 26-09-2013 |
| | | | EP | 2681709 A2 | 08-01-2014 |
| | | | US | 2012231959 A1 | 13-09-2012 |
| | | | WO | 2012122127 A2 | 13-09-2012 |

EPO FORM P0459

For more details about this annex : see Official Journal of the European Patent Office, No. 12/82

**EP 3 790 015 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 382358